# EUROPEAN PATENT APPLICATION

(11) **EP 4 789 681 A1**
(43) Date of publication of application: **12.08.2026**
(21) Application number: 25876085.9
(22) Date of filing: 28.08.2025
(51) Int. Cl.: A61K 39/29, A61K 39/385, A61P 31/20, A61P 35/00, C07K 19/00, C12N 15/62, C12N 15/63, C12N 5/10

(54) **GLYCOSYLATED FUSION PROTEIN, NUCLEIC ACID MOLECULE, EXPRESSION VECTOR, HOST CELL AND USE THEREOF**

(30) Priority: 20.12.2024 CN 202411894669
(71) Applicant: Chimigen Biomedical (Chengdu) Co., Ltd., Chengdu, Sichuan 610219 (CN)
(72) Inventor: SHI, Yuenian, Roslyn Heights, NY 11577 (US); YU, Bo, Kendall Park, NJ 08824 (US); ZHANG, Bo, Chengdu, Sichuan 610219 (CN)
(74) Representative: Angerhausen, Christoph
(86) International application number: PCT/CN2025/117662
(87) International publication number: WO 2026/129723

(57) **Abstract**

The present application provides a glycosyl-modified fusion protein, a nucleic acid molecule, an expression vector, a host cell and applications thereof. A first aspect of the present application provides a glycosyl-modified fusion protein, including a murine Fc variant and a hepatitis B virus antigen, where the murine Fc variant is obtained by subjecting a murine Fc fragment to amino acid mutation and non-mammalian glycosylation modification; the murine Fc variant includes at least one of alanine at position 223, alanine at position 228, alanine at position 230, leucine at position 330, and glutamic acid at position 332; the glycosylation modification does not include sialic acid modification; the numbering of position is performed according to EU numbering system. The fusion protein is capable of binding to and activating DCs and promoting proliferation and activation of specific T cells.

## Description

This application claims priority of Chinese Patent Application No. 202411894669.X, filed with the China National Intellectual Property Administration on December 20, 2024, and entitle with "GLYCOSYL-MODIFIED FUSION PROTEIN, NUCLEIC ACID MOLECULE, EXPRESSION VECTOR, HOST CELL AND APPLICATION THEREOF", which is hereby incorporated by reference in its entirety.

### TECHNICAL FIELD

The present application relates to a glycosyl-modified fusion protein, a nucleic acid molecule, an expression vector, a host cell and application thereof, and pertains to the field of biopharmaceutical technology.

### BACKGROUND

Human hepatitis B virus (HBV) is a member of the family of DNA viruses that primarily infect the liver. HBV mainly infects hepatocytes and can cause acute and chronic liver disease, leading to liver cirrhosis and hepatocellular carcinoma. Approximately 90% of adult individuals infected with HBV can clear the infection, while the remaining 10% become chronic viral carriers, who are at high risk of developing liver cirrhosis and hepatocellular carcinoma (Block, 2016). According to the World Health Organization, the number of people infected with HBV has been more than approximately 2 billion, among whom 350 million are person with chronic HBV. Prophylactic vaccines based on HBV surface antigen (HBsAg) are highly effective in providing protective immunity against HBV infection. These vaccines are developed from HBsAg purified from the plasma of chronic HBV carriers and produced by recombinant DNA technology and synthetic peptides (see U.S. Patents 4,599,230 and 4,599,231). Although these vaccines are highly effective in preventing infection, they are ineffective in eradicating established chronic infection.

When a healthy host (human or animal) encounters an antigen (such as a protein derived from bacteria, viruses, and/or parasites), the host typically triggers an immune response. This immune response may be humoral and/or cellular. In the humoral response, B cells produce antibodies in response to antigen stimulation and secrete them into the blood and/or lymph. The antibodies then neutralize antigens (e.g., viruses) by specifically binding to the antigens on their surfaces, and kill them through phagocytosis by phagocytes and/or complement-mediated mechanisms. The cellular response is characterized by the selection and increase of specific helper cells and cytotoxic T lymphocytes to directly eliminate antigen-containing cells. However, in many individuals, the immune system fails to respond to certain antigens. When an antigen does not stimulate the production of specific antibodies and/or cytotoxic T cells, the immune system is unable to prevent the resulting disease. Consequently, infectious source (e.g., viruses) can establish chronic infections, and the host immune system develops antigen tolerance to the virus. The most common cases of chronic viral infections include hepatitis B, hepatitis C, human immunodeficiency virus, and herpes simplex virus.

In a chronic infection state, the virus can evade the host immune system. Viral antigens are recognized as "self" substances of the body and are therefore not recognized by antigen-presenting cells. The manner in which antigen-presenting cells process encountered antigens depends on the location of the antigen (Steinman et al., 1999). Exogenous antigens are processed in endosomes of APC, and the resulting peptide fragments are mixed with major histocompatibility complex (MHC) class II molecules and are presented on the cell surface. This complex is presented to CD4+ T cells and stimulates the generation of CD4+ T helper cells. Therefore, cytokines secreted by helper cells stimulate B cells to produce antibodies against exogenous antigens (humoral response). Immunization with antigens typically induces an antibody response via this endosomal antigen processing pathway. On the other hand, intracellular antigens are processed in the proteasome, and the resulting peptide fragments form complexes with MHC class I molecules, which are presented on the surface of APCs. Upon binding of this complex to the co-receptor CD8 molecule, the antigen is presented to CD8⁺ T cells, triggering a cytotoxic T lymphocyte (CTL) immune response, thereby killing antigen-bearing host cells.

In patients with chronic viral infections, since viral replication is active, the production of viral antigens will be within host cells and the secreted antigens are present in the humoral circulation. For example, virions, HBV surface antigen, and core antigen (e antigen) can be detected in the blood of chronic HBV carriers. Effective therapeutic vaccines may be capable of inducing CTL responses against intracellular antigens or antigens delivered to appropriate cellular compartments, so as to activate the MHC class I processing pathway. Therapeutic vaccines capable of inducing CTL responses can be processed via the proteasome pathway and presented through the MHC class I pathway (Larsson et al., 2001). This can be achieved by producing antigens within host cells, or the vaccine can be delivered to a suitable cellular compartment for processing and presentation to trigger a cellular response. Several methods for the intracellular delivery of antigens have been described in the literature.

Dendritic cells (DCs) derived from blood monocytes can serve as an immune modulator that stimulates the response of naive T cell due to their ability to act as professional antigen-presenting cells (Steinman et al., 1999; Banchereau and Steinman, 1998). This unique property of dendritic cells in capturing, processing and presenting antigens as well as stimulating naive T cells makes them a very important tool for the development of therapeutic vaccines (Laupeze et al., 1999). Targeting antigen to dendritic cells is a critical step in antigen presentation, and the presence of several receptors of dendritic cells for the Fc region of monoclonal antibodies has been used for this purpose (Regnault et al., 1999). Examples of such approach include the ovarian cancer antibody Mab-B43.13, anti-PSA antibodies, and anti-HBV antibodies-antigen complexes (Wen et al., 1999). Studies have demonstrated that cancer immunotherapy using tumor-associated antigen-loaded dendritic cells generates tumor-specific immune responses and anti-tumor activity (Campton et al., 2000; Fong and Engleman, 2000). Satisfactory results have been obtained in in vivo clinical trials using tumor antigen-pulsed dendritic cells (Tarte and Klein, 1999). These studies clearly demonstrate the efficacy of using dendritic cells to generate immune responses against cancer antigens.

Furthermore, studies have shown that chimeric antigens (fusion of Fc antibody fragment and HBV antigen) can effectively trigger antigen-specific T cell- and B cell-mediated immune responses against HBV and can also break tolerance to HBV antigens under chronic conditions (George et al., U.S. Patent Nos. 8007805B2, 8025873B2, 8029803B2, and 8465745B2; Chinese Patent No. 200480022747.1; Indian Patent No. 225281; Japanese Patent No. 5200201; Korean Patent No. 10-1327719; New Zealand Patent No. 545048; Singapore

Patent No. 119567; South African Patent No. 2006-0804; Australian Patent No. 2012200998; European Patent in force: German, French, and British Patent No. 1664270; Ma et al., 2020; George et al., 2020). Compared to the reports on the engineering of human immunoglobulin Fc fragments, there are relatively few reports on the engineering of mouse immunoglobulin Fc fragments. Regarding whether engineering can enhance the presentation effect or not, we believe it is necessary to conduct research on the engineering of the Fc fragment of mouse immunoglobulin.

### SUMMARY

The present application provides a glycosyl-modified fusion protein, including a murine Fc variant and a hepatitis B virus antigen, where the murine Fc variant is obtained by modifying a murine IgG1 Fc fragment. The fusion protein is capable of binding to DCs, activating DCs, and promoting the proliferation and activation of specific T cells.

The present application also provides a nucleic acid molecule encoding the aforementioned fusion protein, as well as an expression vector and a host cell including the nucleic acid molecule.

The present application further provides a use of the aforementioned fusion protein in the preparation of a medicament for a treatment of a hepatitis B virus.

A first aspect of the present application provides a glycosyl-modified fusion protein, including a murine Fc variant and a hepatitis B virus antigen, where the fusion protein binds to and activates DCs to promote proliferation and activation of specific T cells;
the murine Fc variant is obtained by subjecting a murine Fc fragment to an amino acid mutation and a glycosylation modification;
the murine Fc variant includes at least one of alanine at position 223, alanine at position 228, alanine at position 230, leucine at position 330, and glutamic acid at position 332;
in the glycosylation modification, glycosyl is derived from at least one of mannose, N-acetylglucosamine, and fucose;
in the glycosylation modification, a saccharide chain structure formed by linkage of the glycosyl is selected from at least one of a high-mannose type, an oligomannose type, and a fucose type;
the non-mammalian glycosylation modification does not include sialic acid modification;
a position numbering of amino acid is performed according to EU numbering system.

In the present application, the aforementioned murine Fc variant is obtained by the amino acid mutation and the non-mammalian glycosylation modification on the basis of the wild-type Fc fragment shown in SEQ ID NO: 1.

In addition, the wild-type murine Fc fragment shown in SEQ ID NO:1 is the Fc fragment of murine IgG1, and Fc fragments of other murine IgG than murine IgG1 are also applicable for the present application. For example, when based on the Fc fragment of murine IgG2, the Fc variant obtained by amino acid mutation and non-mammalian glycosylation modification exhibits similar effects of binding to and activating DCs, and promoting the proliferation and activation of specific T cells.

In some specific embodiments, the amino acid sequence of the murine Fc variant is shown in SEQ ID NO: 2 or SEQ ID NO: 3.

The amino acid sequence shown in SEQ ID NO:2 includes 232 amino acid residues, the mutation position is numbered according to the EU numbering system, and the alanine at positions 223 and 228, as well as position 230 are located successively at the underlined positions in the following sequence:

VDKKIVPADCGCAPCICAVPEVSSVFIFPPKPKDVLTITLTPKVTCVVVDISK
DDPEVQFSWFVDDVEVHTAQTQPREEQFNSTFRSVSELPIMHQDWLNGKEFKCRVNS
AAFPAPIEKTISKTKGRPKAPQVYTIPPPKEQMAKDKVSLTCMITDFFPEDITVEWQWN
GQPAENYKNTQPIMDTDGSYFVYSKLNVQKSNWEAGNTFTCSVLHEGLHNHHTEKS
LSHSPGL.

The alanines at positions 223 and 228, as well as positions 230, 330, and 332 shown in SEQ ID NO: 3 are located successively at the underlined positions in the following sequence:

VDKKIVPADCGCAPCICAVPEVSSVFIFPPKPKDVLTITLTPKVTCVVVDISK
DDPEVQFSWFVDDVEVHTAQTQPREEQFNSTFRSVSELPIMHQDWLNGKEFKCRVNS
AAFPLPEEKTISKTKGRPKAPQVYTIPPPKEQMAKDKVSLTCMITDFFPEDITVEWQW
NGQPAENYKNTQPIMDTDGSYFVYSKLNVQKSNWEAGNTFTCSVLHEGLHNHHTEK
SLSHSPGL.

The difference between non-mammalian glycosylation and mammalian glycosylation lies in the presence or absence of sialic acid modification and the content of mannose. In the technical solution provided in this application, glycosylation is mainly N-glycosylation, and the glycosyl is derived from at least one of mannose, N-acetylglucosamine, and fucose; the saccharide chain structure formed by glycosyl linkage is selected from one or more of a high-mannose type, an oligomannose type, and a fucose type. The high-mannose type structure consists of GlcNAc and mannose, and contains 5-9 mannose residues; the oligomannose type structure refers to those fewer than 5 mannose residues; and the fucose type structure refers to containing fucose. In the solution provided by the present application, the aforementioned glycosylation sites are all located on the amino acid at position 297 of the Fc variant.

Further, the non-mammalian is an insect. Further, the non-mammalian is *Spodoptera frugiperda*. Still further, the aforementioned fusion protein is obtained by expression in Sf9 insect cells.

In the present application, hepatitis B virus antigen refers to a type of protein on the surface of the hepatitis B virus (HBV) and is one of the important markers of HBV infection. These surface antigens, i.e., large protein (S1/S2/S), middle protein (S2/S), and small protein (S), are believed to be involved in the binding of the virus to cellular receptor for uptake. The HBV core protein (HBV Core) forms the capsid enclosing the partially double-stranded DNA genome. In the present application, the hepatitis B virus antigen may be HBV S1 and/or S2 and/or CORE (core antigen). Further, in some specific embodiments of the present application, the hepatitis B virus antigen is HBV S1/S2/CORE. HBV S1/S2/CORE, also referred to as the "HBV S1/S2/core protein chimeric antigen", is a fusion of the dominant B-cell epitopes of pre-S1 (preS1) and pre-S2 (preS2) proteins of the hepatitis B virus surface antigen with the hepatitis B virus core antigen (HBcAg).

In the present application, the aforementioned fusion protein mainly includes a hepatitis B virus antigen capable of inducing an immune response in the body and a murine Fc variant that binds to DCs. The murine Fc variant may be linked to the C-terminus of the hepatitis B virus antigen, or, the hepatitis B virus antigen and the murine Fc variant may be linked via a linker peptide. In one embodiment, the amino acid sequence of the linker peptide is at least one of GGGS (SEQ ID NO: 13), SRGGGS (SEQ ID NO: 14), and VRPQGGGS (SEQ ID NO: 15).

In the solution provided by the present application, the aforementioned fusion protein may further include a protein tag to facilitate the expression, detection, and purification of a target gene. Further, the protein tag may be a His-tag, which is attached to the N-terminus of the polypeptide antigen to enhance the purification efficiency of the fusion protein.

The fusion protein as described above is a glycosylated fusion protein including a hepatitis B virus antigen capable of causing an immune response in human and a murine Fc variant. The aforementioned fusion protein can be obtained by expression in insect cells through a baculovirus expression system.

A second aspect of the present application provides a nucleic acid molecule encoding any of the aforementioned fusion proteins.

A third aspect of the present application provides a recombinant expression vector including the aforementioned nucleic acid molecule.

A fourth aspect of the present application provides a host cell including the aforementioned recombinant expression vector.

A fifth aspect of the present application provides a pharmaceutical composition including any of the aforementioned fusion proteins and a pharmaceutically acceptable carrier.

In the present application, the pharmaceutically acceptable carrier is non-toxic to cells or individuals at the dosage and concentrations used. Typically, a physiologically acceptable carrier is a pH-buffer solution. The pharmaceutical composition provided by the present application is further in the form of an injection, where the aforementioned carrier may also include a diluent such as water, ethanol, as well as polyethylene glycol, and an isotonic additive such as sodium chloride, glucose, or glycerol. Additionally, conventional cosolvent, buffer agent, and the like may also be added.

A sixth aspect of the present application provides a use of any one of the aforementioned fusion proteins in the preparation of a medicament for treating hepatitis B virus.

A seventh aspect of the present application provides a use of any one of the aforementioned fusion proteins in the preparation of a medicament for treating any hepatitis B virus expressing HBV S1 and/or S2 and/or core protein antigens.

In the present application, the medicament for treating hepatitis B virus may be an HBV antiviral preparation. In some examples, the HBV antiviral preparation may be a pharmaceutically acceptable agent including one or more compounds selected from tenofovir, tenofovir disoproxil fumarate, tenofovir alafenamide, entecavir, tibuvirin, adefovir dipivoxil, and lamivudine. In other examples, the HBV antiviral preparation is an HBV antiviral RNAi agent that inhibits HBV replication. In still other examples, the HBV antiviral preparation is a core-capsid assembly inhibitor. In yet other examples, the HBV antiviral preparation is a TLR agonist.

In other embodiments of the method, the method further includes administering an immunomodulator to a host. In some examples, the immunomodulator is interferon, such as pegylated interferon α2-a. In other examples, the immunomodulator is an immune checkpoint inhibitor, such as a PD1/PDL1 inhibitor.

In yet other embodiments of the method, the host is a human subject. In other embodiments, the viral infection is HBV infection.

The fusion protein provided by the present application is formed by fusing a murine Fc variant with a hepatitis B virus antigen. The fusion protein can bind to and activate DCs, and promote the proliferation and activation of specific T cells, thereby achieving the therapeutic effect against hepatitis B virus infection in the host.

### BRIEF DESCRIPTION OF DRAWINGS

FIG. 1a shows an SDS-PAGE identification result of Seq2-Sf9, where M1 is a protein molecular weight marker and BSA is a protein standard.
FIG. 1b shows an SDS-PAGE identification result of Seq3-Sf9, where M is a protein molecular weight marker and BSA is a protein standard.
FIG. 2a shows a BLI assay result of Seq2-Sf9 and the FcγRIIA protein.
FIG. 2b shows a BLI assay result of Seq3-Sf9 and FcγRIIA.
FIG. 3 shows an assay result of binding force of Seq2-293 and Seq3-293 to DC2.4 at different concentrations.
FIG. 4a shows an SDS-PAGE/PageBlue staining test result of Seq4-Sf9.
FIG. 4b shows a Western blot test result ofSeq4-Sf9, with the primary antibody being a His tag polyclonal antibody (Rabbit) and the secondary antibody being HRP-conjugated Affinipure Goat Anti-Rabbit IgG(H+L).
FIG. 4c shows an SDS-PAGE/PageBlue staining test result of Seq5-Sf9 and Seq6-Sf9.
FIG. 4d shows a Western blot test result of Seq5-Sf9 and Seq6-Sf9, with the primary antibody being His-tag polyclonal antibody and the secondary antibody being HRP-conjugated Affinipure Goat Anti-Rabbit IgG (H+L).
FIG. 4e shows an N-glycosylation modification result at position 484 of Seq4-Sf9 (position 297 of the Fc fragment).
FIG. 4f shows a percentage result of fluorescence intensity detected by a flow cytometry for Seq5-Sf9 and Seq6-Sf9.
FIG. 5a shows an SDS-PAGE/PageBlue staining test result of Seq7-Sf9, with Lane 1 being a sample eluted from Ni column; Lane 2 being a sample dialyzed into a storage solution.
FIG. 5b shows a WB result of Seq7-Sf9, with the antibody being goat anti-mouse IgG(H+L), Lane 1 being the sample eluted from the Ni column and Lane 2 being the sample dialyzed into the storage solution.
FIG. 5c shows an assay result of binding force of Seq7-Sf9 to mouse dendritic cell line DC2.4, with Mouse IgG1 serving as a control protein.
FIG. 6a shows an expression detection result of CD54 on the cell surface after DCs are loaded with Seq7-Sf9 fusion protein for 48 hours, where Donor #2, Donor #4, and Donor #5 represent three PBMC volunteers, respectively.
FIG. 6b shows an expression detection result of CD83 on the cell surface after DCs are loaded with Seq7-Sf9 fusion protein for 48 hours, where Donor #2, Donor #4, and Donor #5 represent three PBMC volunteers, respectively.
FIG. 6c shows an expression detection result of CD86 on the cell surface after DCs are loaded with Seq7-Sf9 fusion protein for 48 hours, where Donor #2, Donor #4, and Donor #5 represent three PBMC volunteers, respectively.
FIG. 6d shows a result of proliferation assay for CD4+ T cells after DCs are loaded with Seq7-Sf9, where Donor #2, Donor #4, and Donor #5 represent three PBMC volunteers, respectively.
FIG. 7 shows serum titer of rabbit immunized with Seq7-Sf9.
FIG. 8 shows an SDS-PAGE gel identification result of a fusion protein Seq8-293, where M1 represents a protein molecular weight marker, R represents a reduced protein, and NR represents a non-reduced protein.
FIG. 9 shows an SDS-PAGE gel identification result of a fusion protein Seq8-Sf9.
FIG. 10 shows an SDS-PAGE gel identification result of a fusion protein Seq9-Sf9.
FIG. 11a shows an expression detection result of CD54 on the cell surface after DCs are loaded with Seq9-Sf9 and Seq10-293 for 48 hours.
FIG. 11b shows an expression detection result of CD83 on the cell surface after DCs are loaded with Seq9-Sf9 and Seq10-293 for 48 hours.
FIG. 12a shows an expression detection result of CD54 on the cell surface after DCs are loaded with Seq8-Sf9 for 48 hours.
FIG. 12b shows an expression detection result of CD83 on the cell surface after DCs are loaded with Seq8-Sf9 for 48 hours.
FIG. 13a shows an expression detection result of CD54 on the cell surface after DCs are loaded with Seq8-Sf9 and Seq8-293.
FIG. 13b shows an expression detection result of CD83 on the cell surface after DCs are loaded with Seq8-Sf9 and Seq8-293.
FIG. 14a shows an expression detection result of CD54 on the cell surface after DCs are loaded with Seq8-Sf9 and Provenge.
FIG. 14b shows an expression detection result of CD83 on the cell surface after DCs are loaded with Seq8-Sf9 and Provenge.
FIG. 15a shows a proliferation detection result of CD4+T cells from volunteer 1 after DCs are loaded with Seq8-Sf9 and Provenge.
FIG. 15b shows a proliferation detection result of CD4+T cells from volunteer 2 after DCs are loaded with Seq8-Sf9 and Provenge.
FIG. 16 shows results of IFN-γ secreted by CD8+ T cells activated by DCs loaded with Seq8-Sf9, detected by ELISPOT.
FIG. 17 shows protein expression results of PAP in the human prostate cancer cell lines PC3 and LNCap, detected by Western Blot.
FIG. 18a shows a detection result of killing effect of CTL induced by DCs loaded with Seq8-Sf9 and Provenge on PC3 tumor cells.
FIG. 18b shows a detection result of killing effect of CTL induced by DCs loaded with Seq8-Sf9 and Provenge on LNCap tumor cells.
FIG. 19a shows a titer detection result of anti-Seq6-Sf9 serum antibody of SD rats immunized by subcutaneous injection of Seq8-Sf9 (with doses of 0/2/10/50 ug), where the samples are collected at time points Day 0 (before a first injection), Day 14 (before a second injection), Day 28 (before a third injection) and Day 35 (one week after the third injection), n = 6.
FIG. 19b shows a titer detection result of serum antibody of SD rats immunized by subcutaneous injection of Seq8-Sf9 (with doses of 0/2/10/50 ug) on Day 14.
FIG. 19c shows a titer detection result of serum antibody of SD rats immunized by subcutaneous injection of Seq8-Sf9 (with doses of 0/2/10/50 ug) on Day 28.
FIG. 19d shows a titer detection result of serum antibody of SD rats immunized by subcutaneous injection of Seq8-Sf9 (with doses of 0/2/10/50 ug) on Day 35.
FIG. 20a shows a result of IFN-γ secreted by spleen cells of SD rats on Day 35 after immunization with Seq8-Sf9, detected by ELISPOT.
FIG. 20b is a spot diagram of IFN-γ secreted by spleen cells of SD rats on Day 35 after immunization with Seq8-Sf9, detected by ELISPOT.
FIG. 21 is a HE pathological staining image of prostate tissue of SD rats on Day 35 after immunization with Seq8-Sf9 (20× magnification).
FIG. 22a shows comparison of expression detection results of CD54 on the cell surface after DCs are each loaded with Seq9-Sf9 and Seq8-Sf9 for 48 hours.
FIG. 22b shows comparison of expression detection results of CD83 on the cell surface after DCs are each loaded with Seq9-Sf9 and Seq8-Sf9 for 48 hours.

### DESCRIPTION OF EMBODIMENTS

To enable those skilled in the art to better understand the solution of the present application, the present application is described in further detail below. The specific embodiments set forth below are merely for describing the principles and features of the present application, and the examples cited are only used to explain the present application, not to limit the scope of the present application. Based on the examples of the present application, all other embodiments obtained by persons of ordinary skill in the art without creative efforts shall fall within the scope of protection of the present application.

### Example 1 Affinity detection of Fc variant

### 1.1 Acquisition and affinity detection of Fc variants expressed in Sf9 insect cells

To enhance the stability of the mouse IgG Fc fragment, mutations are performed at three positions 223, 228, and 230 of Fc fragment, which is based on the wild-type murine Fc fragment shown in SEQ ID NO: 1. The amino acid sequence after mutation is shown in SEQ ID NO: 2. To enhance the binding of the mouse IgG1 Fc fragment to DCs and DCs activation, mutations are performed at five positions 223, 228, 230, 330, and 332 of Fc fragment, which is based on the wild-type murine Fc fragment. The amino acid sequence after mutation is shown in SEQ ID NO: 3.

The signal sequence derived from the gp64 protein of *Autographa californica* multiple nucleopolyhedrovirus (AcMNPV) (amino acid sequence shown in SEQ ID NO: 11) is cloned into the pFastBacHTa (Thermo Fisher Scientific; Cat# 10584-027) vector digested with RsrII (New England Biolabs, R051S), obtaining pFastBacHTa-gp64. The signal sequence derived from the gp67 protein of AcMNPV (amino acid sequence shown in SEQ ID NO: 12) is cloned into the pFastBacHTa (Thermo Fisher Scientific; Cat# 10584-027) vector digested with RsrII (New England Biolabs, R051S), obtaining pFastBacHTa-gp67. DNA sequence corresponding to the amino acid shown in SEQ ID NO: 2 or SEQ ID NO: 3 is synthesized, and the fragment is subcloned into the pFastBacHTa-gp64 vector digested with Sal I (New England Biolabs, R3138S) and Hind III (New England Biolabs, R0104S) for expression in insect cells. The recombinant plasmid is transformed into DH10Bac competent cells, followed by culturing on a fresh LB agar plate containing 50 µg/mL kanamycin, 7 µg/mL gentamicin, 10 µg/mL tetracycline, 100 µg/mL Bluo-gal, and 40 µg/mL IPTG. After overnight incubation, white colonies are selected, and recombinant bacmid DNA is isolated according to standard protocols. The positive bacmid is transiently transfected into 2 mL Sf9 insect cells through a transfection reagent. Transfected cells are incubated in ESF 921 medium for a period of time, and the cells and supernatant are collected. Proteins are purified from the supernatant by Protein A and designated Seq2-Sf9 and Seq3-Sf9, respectively.

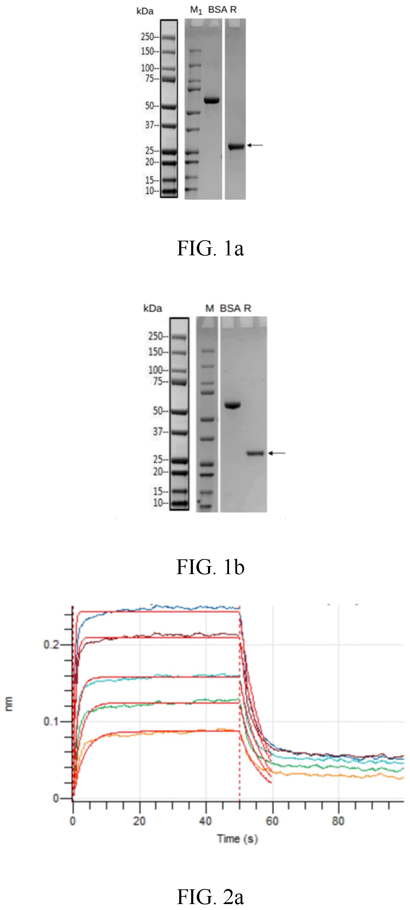

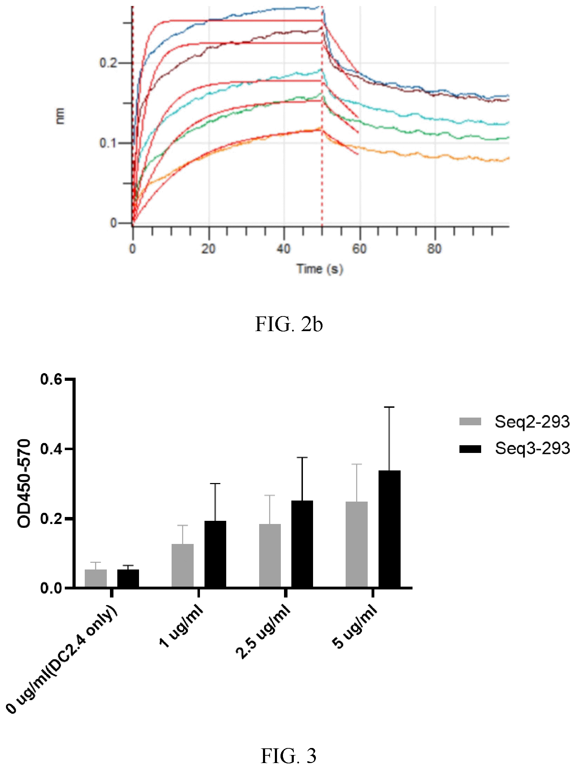

Seq2-Sf9 and Seq3-Sf9 are identified by Coomassie blue-stained SDS-PAGE gel, with BSA as the control protein. The identification results are shown in FIGS. 1a and 1b, respectively. The positions indicated by the arrows correspond to Seq2-Sf9 and Seq3-Sf9, confirming that purified protein with a molecular weight of approximately 26 kDa is obtained. Multi-concentration affinity assay of purified Seq2-Sf9 and Seq3-Sf9 proteins is performed using biolayer interferometry (BLI). A concentration gradient of 5000 nM, 2500 nM, 1250 nM, 625 nM, and 312.5 nM is set to determine the affinity of Seq2-Sf9 and Seq3-Sf9 proteins for FcγRIIA protein. The detection results are shown in FIGS. 2a and 2b, respectively. The curves from bottom to top represent sequentially increasing concentrations of Seq2-Sf9 and Seq3-Sf9 proteins. After calculation, the affinities (KD values) of Seq2-Sf9 and Seq3-Sf9 to FcγRIIA protein are 7.893E-07 and 4.496E-07, respectively. A smaller value indicates higher affinity. It demonstrates that the five mutations in the mouse Fc domain can enhance the affinity for FcγRIIA.

### 1.2 Acquisition of Fc variants expressed by 293 cells and detection of their binding to DC cells

The synthesized DNA sequences corresponding to SEQ ID NO: 2 and SEQ ID NO: 3 are cloned into the eukaryotic expression vector pcDNA3.4 with a secretion signal peptide, with the digestion sites of EcoRI (New England Biolabs, R0101V) and HindIII (New England Biolabs, R0104S). The resulting product is electroporated into *Escherichia coli* Trans5α cells. After screening with ampicillin, the single clone is sequenced to obtain the correct recombinant plasmids. Then, the host bacteria containing the recombinant plasmids are subjected to amplification culture, and sterile endotoxin-free recombinant plasmids are obtained using an endotoxin-removal kit. The sterile endotoxin-free recombinant plasmids are mixed with a polyplus suspension cell transfection reagent and transfected into HEK293F cells. After 5 days of amplification culture in a serum-free medium, the culture supernatant is collected and and separated and purified using Protein A resin to obtain proteins Seq2-293 and Seq3-293.

Seq2-293 and Seq3-293 can enhance binding to FcR, which is mainly expressed on the surface of various mononuclear cells, including dendritic cells (DCs). Since the above Fc variants are derived from the mouse Fc fragment, the mouse dendritic cell line DC2.4 is chosen as the research object, which expresses relatively stable FcR and can bind to the murine Fc. Under normal conditions, Fc exhibits weak affinity for its receptor FcR. Seq2-293 and Seq3-293 at equal concentrations are incubated with DC2.4 cells at 4°C for 1 h. Biotin-labeled anti-mouse IgG1 (anti-Mouse IgG1 Biotin) and streptavidin-HRP (Streptavidin-HRP) are added separately for reaction. Color development is performed using TMB substrate, followed by termination of the reaction. Fc/FcR bound on the surface of DC2.4 cells is indicated by visible light OD450-570 detection.

The detection results are shown in FIG. 3. It can be seen that the binding affinity of Seq3-293 to DC2.4 is significantly higher than that of Seq2-293 at the same concentration, and is proportional to the working concentration. That is, the Fc variant with five mutations can enhance binding to DCs and exhibits considerable potential for antigen presentation.

### Example 2 Preparation of HBV-mFc proteins and evaluation of DC binding effect

### 2.1 Preparation of Seq4-Sf9, Seq5-Sf9, and Seq6-Sf9 proteins

The five-mutation mouse Fc with the amino acid sequence shown in SEQ ID NO: 3 is fused with hepatitis B virus antigen (including HBV S1/S2/Core) via a linker peptide for expression, and a hydrophilic peptide (SEQ ID NO: 16) is linked to its C-terminus to obtain a fusion protein with the amino acid sequence shown in SEQ ID NO: 4, where the amino acids at positions 36-394 correspond to the hepatitis B virus antigen and the amino acids at positions 403-634 correspond to the five-mutation mouse Fc with the amino acid sequence shown in SEQ ID NO: 2.

The three-mutation mouse Fc with the amino acid sequence shown in SEQ ID NO: 2 is fused with the hepatitis B virus antigen (including HBV S1/S2) via a linker peptide for expression, obtaining a fusion protein with the amino acid sequence shown in SEQ ID NO: 5, where the amino acids at positions 36-209 correspond to the hepatitis B virus antigen and the amino acids at positions 218-449 correspond to the three-mutation mouse Fc with the amino acid sequence shown in SEQ ID NO: 2.

The five-mutation mouse Fc with the amino acid sequence shown in SEQ ID NO: 3 is fused with the hepatitis B virus antigen (including HBV S1/S2) via a linker peptide for expression, obtaining a fusion protein with the amino acid sequence shown in SEQ ID NO: 6, where the amino acids at positions 36-209 correspond to the hepatitis B virus antigen and the amino acids at positions 218-449 correspond to the five-mutation mouse Fc with the amino acid sequence shown in SEQ ID NO: 2.

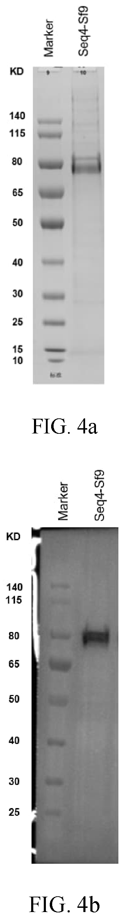

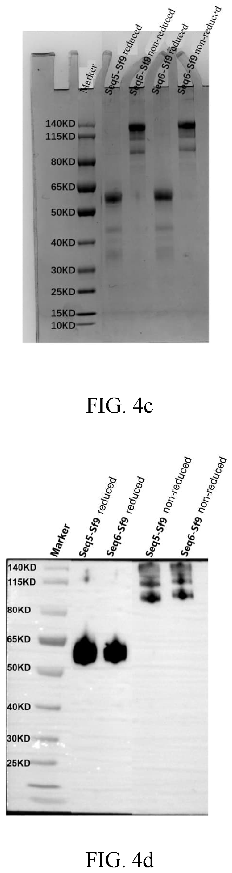

The gene encoding the amino acid sequence shown in SEQ ID NO: 4 is ligated into the pFastBacHTa-gp64 vector digested with Sal I (New England Biolabs, R3138S) and Hind III (New England Biolabs, R0104S) for expression in insect cells. The genes encoding the amino acid sequences shown in SEQ ID NO: 5 and SEQ ID NO: 6 are ligated into the pFastBacHTa-gp67 vectors digested with Sal I (New England Biolabs, R3138S) and Hind III (New England Biolabs, R0104S) respectively, for expression in insect cells. The recombined vector is transformed into *Escherichia coli* DH10Bac for transposition to generate recombinant baculovirus. The recombinant baculovirus is isolated and used to transfect Sf9 insect cells. The transfected insect cells are incubated in ESF921 medium at 27°C for 72 hours, and then cell pellets are collected and lysed by sonication. Fusion proteins are purified from the infected Sf9 cells using the affinity chromatography column and named Seq4-Sf9, Seq5-Sf9, and Seq6-Sf9, respectively. Coomassie blue-stained SDS-PAGE gel and Western blotting are performed for detection, and the results are shown in FIGS. 4a-4d. The apparent molecular weights of the monomers of Seq4-Sf9, Seq5-Sf9, and Seq6-Sf9 fusion proteins are approximately 77 kDa, 57 kDa, and 57 kDa, respectively.

### 2.2 Glycosylation identification of Seq4-Sf9

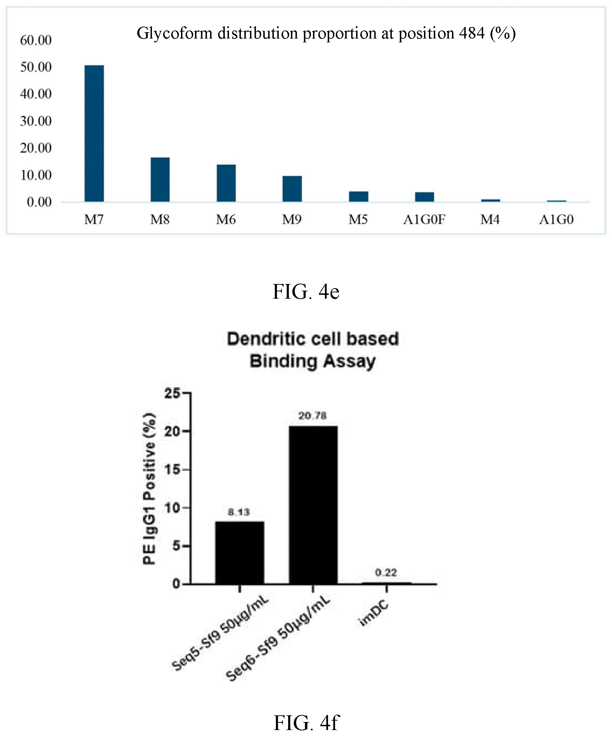

The fusion protein Seq4-Sf9 obtained from the Sf9 insect cell expression system is sequentially treated with Lys-C protease and trypsin, and the digested peptide fragment samples are analyzed by liquid chromatography-mass spectrometry. Raw data obtained by liquid chromatography-mass spectrometry are analyzed using software, and it is confirmed that the ratio of N-glycosylation modification of asparagine at position 484 (position 297 of the Fc fragment) is 68.93%, and the glycoform distribution proportion is shown in FIG. 4e, indicating that glycosylation modification of the fusion protein expressed in the Sf9 insect system is dominated by high-mannose glycoform, accompanied by small amounts of oligomannose and fucose glycoform (glycoform is designated according to the Oxford nomenclature).

### 2.3 Detection of the binding potency of fusion protein HBV-mFc

To evaluate the binding level of the fusion protein HBV-mFc to dendritic cells, imDCs are induced using a classical adherence method. That is, under serum-free conditions, monocytes exhibit certain adhesiveness; non-adherent cells (mainly T cells and B cells) are removed and the remaining cells can be induced to differentiate into immature DCs (immature Dendritic cells, imDCs) in a culture system containing granulocyte-macrophage colony-stimulating factor (GM-CSF) and interleukin-4 (IL-4). The imDCs are incubated with fusion proteins Seq5-Sf9 and Seq6-Sf9 (50 µg/mL each) on ice (2-8°C) for 1 h to allow binding. After gently washing away unbound fractions, the cells are incubated with the secondary antibody PE Rat anti-Mouse IgG1 for a period of time, and then the fluorescence percentage of the fusion proteins Seq5-Sf9 and Seq6-Sf9 bound to DCs is detected by flow cytometry. The murine Fc segment of Seq5-Sf9 contains three mutations, while the murine Fc segment of Seq6-Sf9 contains five mutations. The fluorescence percentage represents the proportion of fusion proteins bound to DCs relative to total cells. As shown by fluorescence intensity percentage detected by flow cytometry in FIG. 4f, the imDC negative control exhibits low fluorescence, indicating that the cell has a low non-specific background. The binding of the five-mutation protein (Seq6-Sf9) to imDCs is significantly higher than that of the triple-mutation protein (Seq5-Sf9).

### 2.4 Evaluation of in vitro activation effect of the protein HBV-mFc

### 2.4.1 Preparation of fusion protein Seq7-Sf9

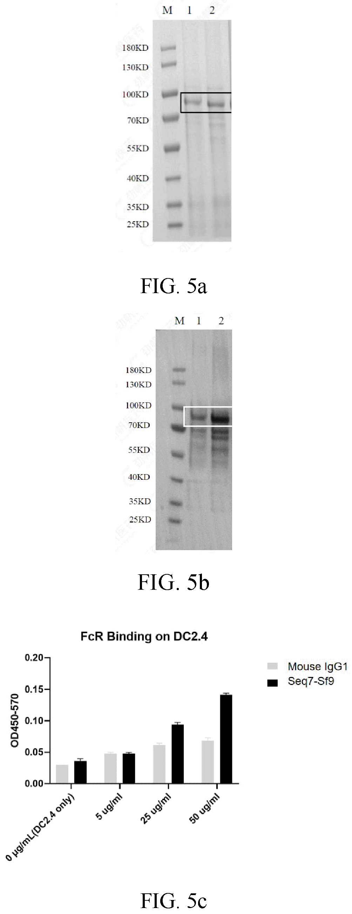

The three-mutation mouse Fc with the amino acid sequence shown in SEQ ID NO: 2 is fused with the hepatitis B virus antigen (including HBV S1/S2/Core) via a linker peptide for expression, and a hydrophilic peptide is linked to the C-terminus, obtaining a fusion protein with the amino acid sequence shown in SEQ ID NO: 7, where amino acids at positions 36-394 correspond to the hepatitis B virus antigen, and amino acids at positions 403-634 correspond to the three-mutation mouse Fc with the amino acid sequence shown in SEQ ID NO: 2. The protein expression method is as described in Section 2.1, using the pFastBacHTa-gp64 vector; and the protein is designated as Seq7-Sf9. Biochemical assay is carried out by Coomassie blue-stained SDS-PAGE gel and Western blotting. Test results are shown in FIGS. 5a-5b. The apparent molecular weight of the monomer of the fusion protein is approximately 77 kDa.

### 2.4.2 Evaluation of the binding force of Seq7-Sf9 to DCs

Seq7-Sf9 at different concentrations (Seq7-Sf9 has a concentration of 0 µg/mL, 5 µg/mL, 25 µg/mL, and 50 µg/mL) is incubated with DC2.4 cells at 4°C for 1 h, followed by the addition of biotin-labeled anti-mouse IgG1 (anti-Mouse IgG1 Biotin) and Streptavidin-HRP for reaction. The reaction is terminated after color development with TMB substrate. The fusion protein bound to the cell surface is indicated by the absorbance value at OD450-570. At the same time, natural mouse IgG1 is used as a control. As shown in FIG. 5c, Seq7-Sf9 exhibits significantly stronger binding potency to DC2.4 than the control mouse IgG1, suggesting that the fusion protein possesses strong potential for antigen presentation.

### 2.4.3 Evaluation of the immune-activation effect of fusion protein Seq7-Sf9 using DCs induced from CD14+ monocytes in healthy human peripheral blood mononuclear cells (PBMCs)

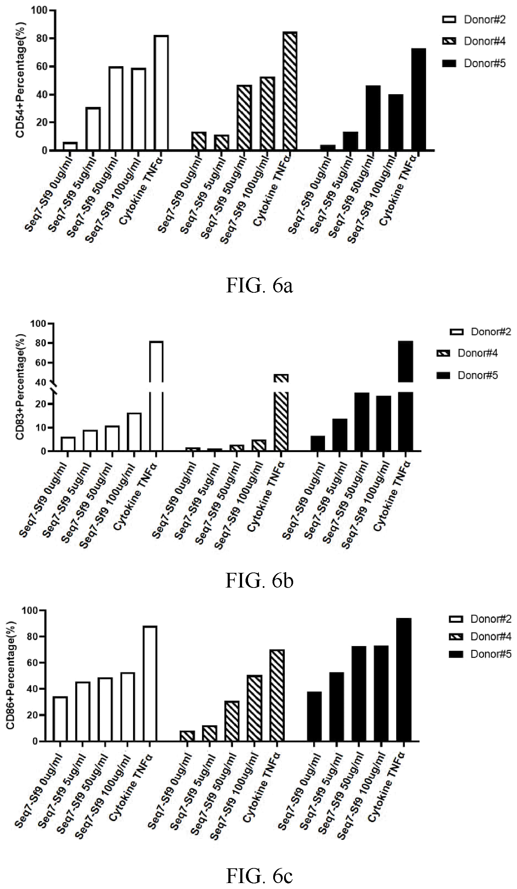

To evaluate the immune-activation effect of fusion protein Seq7-Sf9, CD14+ monocytes are sorted from PBMCs of three healthy human beings and induced to differentiate into immature DCs in a culture system containing granulocyte-macrophage colony-stimulating factor (GM-CSF) and interleukin-4 (IL-4). Immature DCs exhibit strong ability of phagocytosis, and the murine Fc domain contained in the fusion protein would enhance uptake function of immature DCs, promoting the maturation and differentiation of DCs. Flow cytometry is used to detect changes in surface markers of DCs loaded with Seq7-Sf9 fusion protein, including the adhesion molecule CD54 (ICAM-1: Intercellular Adhesion Molecule-1), which facilitates the adhesion and interaction between mature DCs with other immune cells (such as T cells), as well as CD83 involved in antigen presentation and T-cell activation and the co-stimulatory molecule CD86. The test results are shown in FIGS. 6a-6c. In three different volunteers, DCs loaded with fusion protein Seq7-Sf9 exhibit high expression of the maturation markers CD54, CD83, and CD86 on their surfaces, indicating that the fusion protein possesses a strong DC-activating effect. This activates the antigen-presenting capacity of vaccine-loaded DCs, which, as an initiation step, initiates an immune response.

### 2.4.2 In vitro evaluation of key-point for the effect of Seq7-Sf9-loaded DCs on T cell immune response

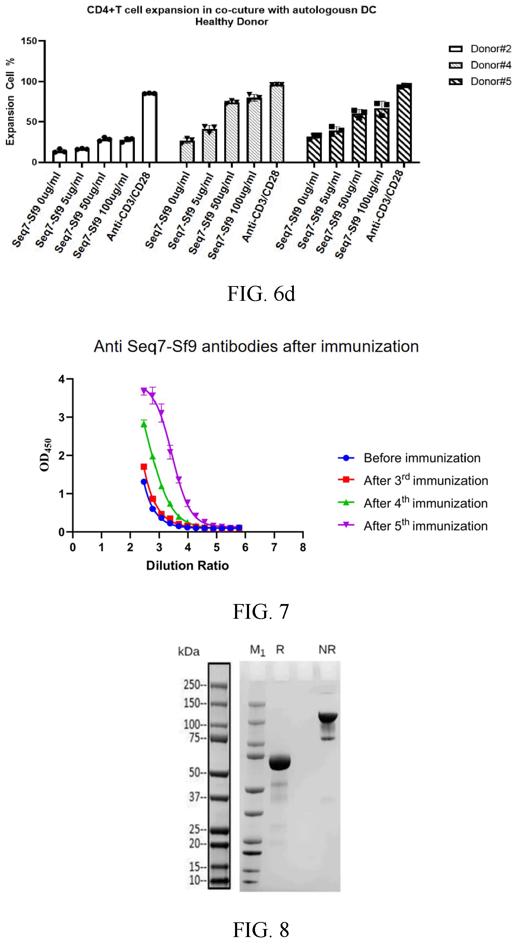

For CD4+ T cell proliferation, the primary function of mature DCs is antigen presentation; antigen peptide/MHC-I complex or antigen peptide/MHC-II complex on DCs surface is recognized by T cell surface receptor (TCR). A critical step for DC-T cell activation is the proliferation of T cells. Therefore, DCs loaded with Seq7-Sf9 are co-cultured with CD4⁺ T cells labeled with CFSE fluorescent dye. After antigen presentation, T cells rapidly proliferate and differentiate into T helper (Th) cells. The immune condition is thus evaluated based on the proliferation of CD4⁺ T cells. The result is shown in FIG. 6d, where, compared with the negative control of CD4⁺ T cells from the same source (0 µg/mL Seq7-Sf9), DCs loaded with Seq7-Sf9 can significantly promote the proliferation of T cells, which is positively correlated with the concentration of the Seq7-Sf9.

### 2.5 Rabbit serum titer assay

Antibody levels in rabbit serum are evaluated by ELISA. Two healthy male New Zealand rabbits aged 3-4 months are selected as experimental animals. 15 µg of the test substance, protein Seq7-Sf9, is dissolved in 0.5-1 mL PBS solution, and fully emulsified at a volume ratio of 1:1 with complete/incomplete Freund's adjuvant (4 mg/mL). The emulsified solution is then injected subcutaneously at four sites on the back, and a total of six immunizations are administrated (D1, D15, D29, D43, D64, D92), with an interval of 2-4 weeks between each immunization. The immunization dosage is 15 µg per rabbit per immunization. (Complete Freund's adjuvant is used for the first immunization; incomplete Freund's adjuvant is used for the 2nd, 3rd, 4th, 5th, and 6th immunizations.)

Plates are coated with protein Seq7-Sf9 (200 ng per well) and incubated overnight, then blocked with PBS +5% (w/v) BSA for 2 hours. These plates are incubated with serially diluted serum samples for one hour (each serum sample is diluted in a 2-fold series starting at a dilution of 1: 300, with a total of 12 dilutions). The binding between Seq7-Sf9 and IgG is detected using goat anti-rabbit IgG antibody. The plates are developed using a TMB (3,3',5,5'-tetramethylbenzidine) two-component peroxidase substrate kit (KPL), and the reaction is terminated by adding a stopping solution. Absorbance at 450 nm is immediately recorded using a SpectraMax Plus spectrophotometer (Molecular Devices) (FIG. 7). The results show that antibody levels increase after persistent infection of rabbits with Seq7-Sf9.

### Example 3 Preparation and bioactivity assessment of Seq8-293, Seq8-Sf9, and Seq9-Sf9

### 3.1 Preparation of Seq8-293

The three-mutation mouse Fc with the amino acid sequence shown in SEQ ID NO: 2 is fused with the prostate cancer-specific marker PAP via a linker peptide for expression, obtaining a fusion protein with the amino acid sequence shown in SEQ ID NO: 8, where the amino acids at positions 1-354 correspond to the prostate cancer-specific marker PAP and the amino acids at positions 361-592 correspond to the triple-mutant mouse Fc with the amino acid sequence shown in SEQ ID NO: 2.

The gene encoding the fusion protein with the amino acid sequence shown in SEQ ID NO: 8 is synthesized, and the gene sequence encoding the fusion protein is ligated into an expression vector pcDNA3.4, with the restriction enzyme sites EcoRI (New England Biolabs, R0101V) and HindIII (New England Biolabs, R0104S) to prepare a recombinant expression plasmid. The recombinant expression plasmid is transiently transfected into eukaryotic HD293F cells, followed by cell culture. The cell culture medium is collected, centrifuged, and filtered. The filtered culture supernatant is loaded onto a Protein A affinity chromatography column for purification, obtaining a fusion protein named Seq8-293. Biochemical assay is performed using Coomassie blue-stained SDS-PAGE gel, and the assay results are shown in FIG. 8.

### 3.2 Preparation of Seq8-Sf9

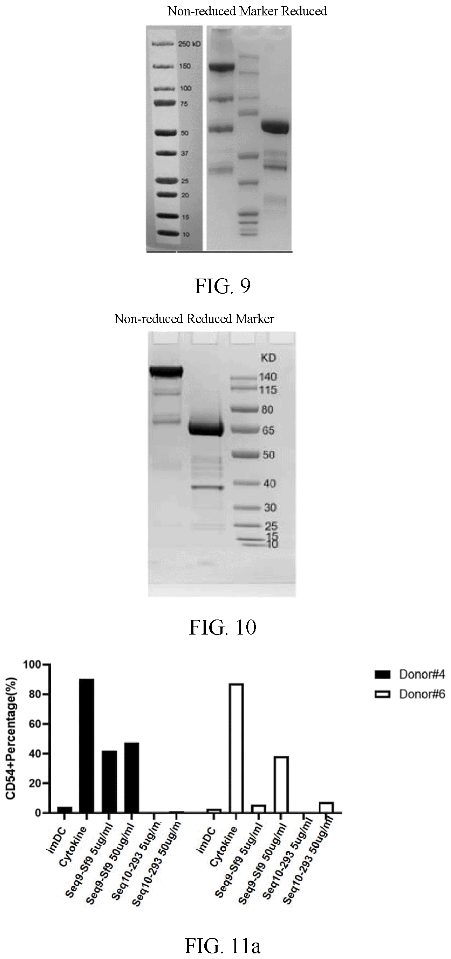

The three-mutation mouse Fc with the amino acid sequence shown in SEQ ID NO: 2 is fused with the prostate cancer-specific marker PAP via a linker peptide for expression, obtaining the fusion protein with the amino acid sequence shown in SEQ ID NO: 8. The corresponding coding sequence is ligated into the pFastBacHTa-gp67 vector digested with Sal I (New England Biolabs, R3138S) and Hind III (New England Biolabs, R0104S) for expression in insect cells. The recombined vector is then transformed into *Escherichia coli* DH10Bac for transposition to generate recombinant baculovirus. The recombinant baculovirus is isolated and used to transfect Sf9 insect cells. The transfected insect cells are incubated in ESF 921 medium at 27 °C for 72 hours to express the target protein. The supernatant is harvested by centrifugation, and the obtained protein is purified by Protein A and named Seq8-Sf9. Biochemical assay is performed using Coomassie blue-stained SDS-PAGE gel, and the detection results are shown in FIG. 9.

### 3.3 Preparation of Seq9-Sf9

The five-mutation mouse Fc with the amino acid sequence shown in SEQ ID NO: 3 is fused with the prostate cancer-specific marker PAP via a linker peptide for expression, obtaining a fusion protein with the amino acid sequence shown in SEQ ID NO: 9, where the amino acids at positions 1-354 correspond to the prostate cancer-specific marker PAP and the amino acids at positions 361-592 correspond to the five-mutation mouse Fc with the amino acid sequence shown in SEQ ID NO: 3.

Using the same method as in Section 3.2, the aforementioned fusion protein is expressed in Sf9 insect cells. The fusion protein obtained by expression is named Seq9-Sf9. Biochemical assay is performed using Coomassie blue-stained SDS-PAGE gel, and the detection results are shown in FIG. 10.

### 3.4 Detection of DC activation effect of Seq9-Sf9

The human wild-type Fc fragment is expressed in mammalian 293 cells together with PAP, a specific marker for prostate cancer, using the same method as in Section 3.1 to obtain a fusion protein Seq10-293 having the amino acid sequence shown in SEQ ID NO: 10. Using the fusion protein Seq10-293 as a control, the effects of the Fc variant and insect glycoform on DC activation are verified.

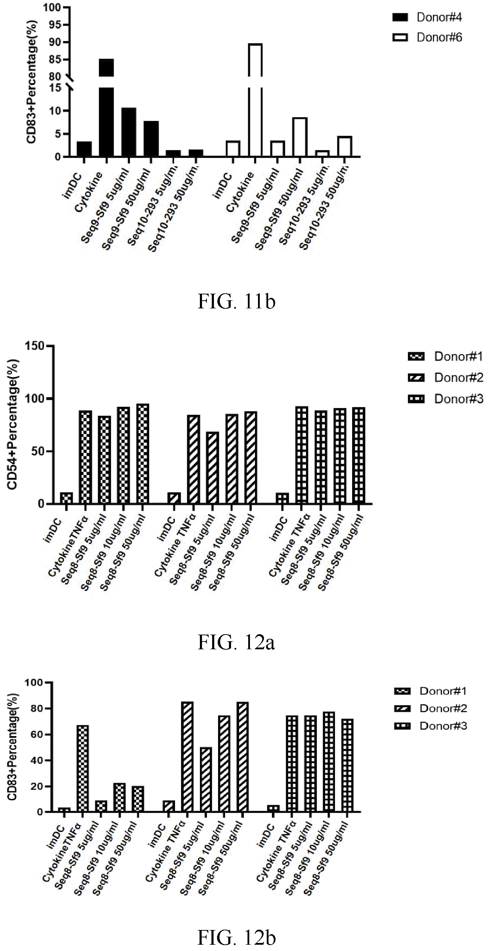

CD14+ monocytes are sorted from PBMCs of two healthy human beings and induced to differentiate into immature DCs in a culture system containing granulocyte-macrophage colony-stimulating factor (GM-CSF) and interleukin-4 (IL-4). The immature DCs possess powerful phagocytic function and are loaded with fusion proteins Seq9-Sf9 and Seq10-293, respectively. DCs phagocytize the fusion proteins and present antigens, thereby activating DCs and expressing activation markers (including CD54 and CD83, etc.). Changes in surface markers of vaccine-loaded DCs are detected by flow cytometry. The detection results are shown in FIGS. 11a-11b. Under the same concentration, Seq9-Sf9 can significantly activate DCs and CD54 and CD83 are highly expressed on the cell surface, compared with Seq10-293. The above results demonstrate that the murine Fc variant and non-mammalian glycoforms in the fusion protein can significantly enhance phagocytosis and activation of DCs, as well as the expression of activation phenotypes, such as CD54 and CD83.

### 3.5 Detection of DC activation effect of Seq8-Sf9

### 3.5.1 Evaluation of the immune activation effects of the fusion protein Seq8-Sf9 using DCs induced from CD14+ monocytes in human peripheral blood PBMCs

To evaluate the immune-activating effects of Seq8-Sf9, CD14+ monocytes are sorted from PBMCs of three healthy human beings and induced to differentiate into immature DCs in a culture system containing granulocyte-macrophage colony-stimulating factor (GM-CSF) and interleukin-4 (IL-4). The immature DCs have powerful phagocytic function, and the murine Fc domain contained in the fusion protein will enhance the uptake function of immature DCs, promoting DC maturation and differentiation. Changes in surface markers of vaccine-loaded DCs are detected by flow cytometry, including the adhesion molecule CD54 (ICAM-1: Intercellular Adhesion Molecule-1), which facilitates the adhesion and interaction between mature DCs and other immune cells (such as T cells), as well as CD83 involved in antigen presentation and T-cell activation. The detection results are shown in FIGS. 12a-12b. In three different volunteers, CD54 and CD83 are highly expressed on the cell surface of DCs loaded with the Seq8-Sf9. It indicates that the fusion protein has a potential DC activation effect, activating the antigen-presenting capacity of Seq8-Sf9-loaded DCs and starting an immune response as an initiation step.

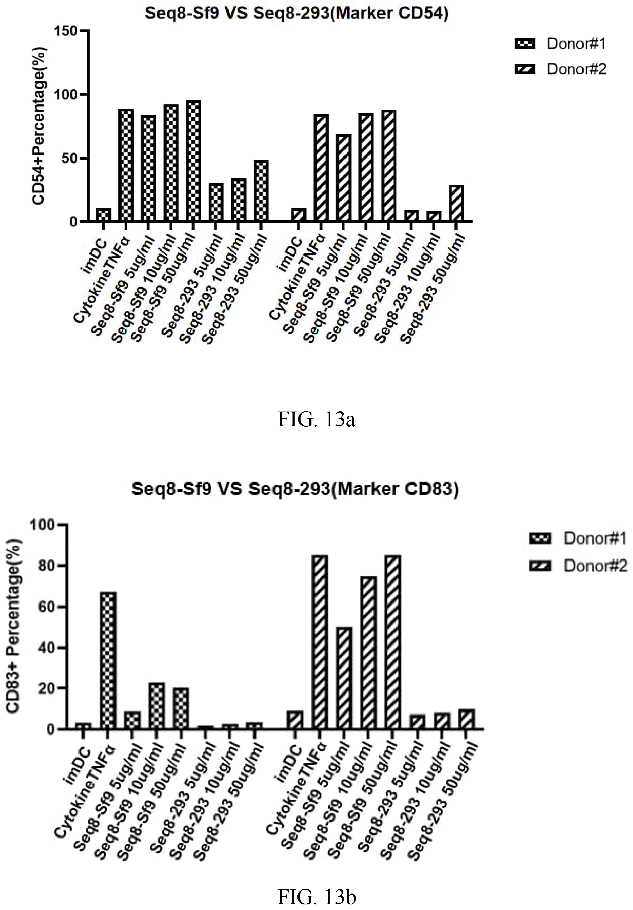

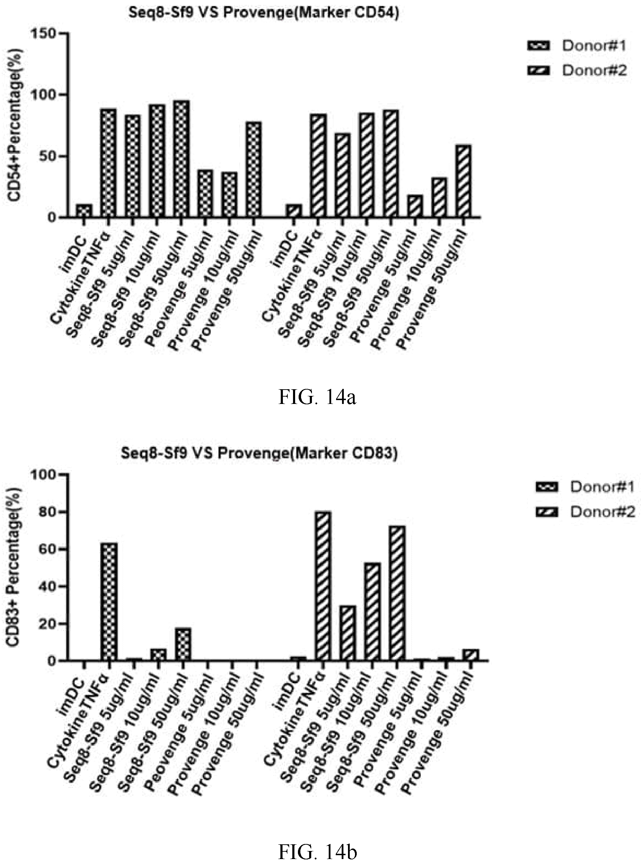

Furthermore, the immune activation effects of Seq8-Sf9, Seq8-293, and the positive control drug Provenge (sipuleucel-T) are compared. The comparison results are shown in FIGS. 13a-13b and 14a-14b; and it can be seen that the Fc variant and non-mammalian glycosylation enhance DC activation effect, and Seq8-Sf9 exhibits a stronger activation ability of DCs.

### 3.5.2 In vitro evaluation of key-point for the effect of Seq8-Sf9-loaded DCs on T cell immune response

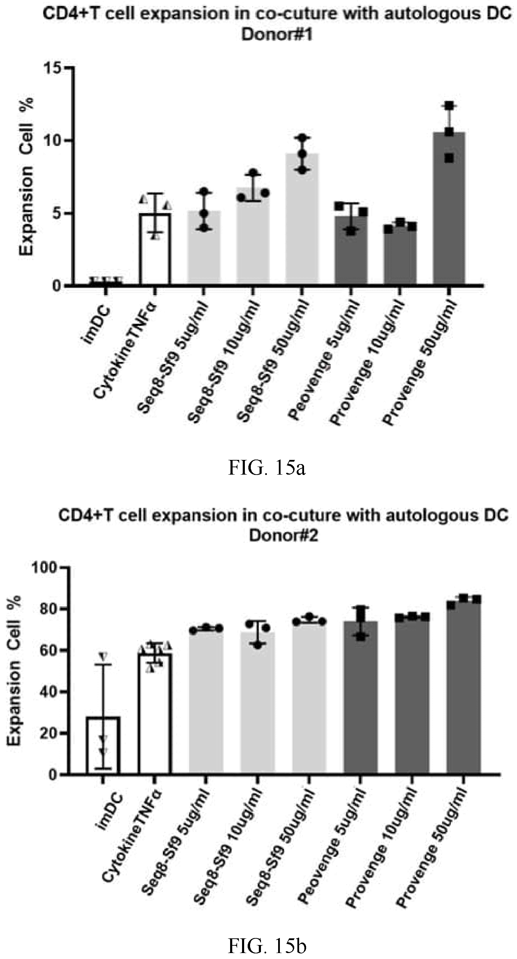

The primary function of mature DCs is antigen presentation for CD4+T cell proliferation. Antigen peptide/MHC-I complex or antigen peptide/MHC-II complex on cell surfaces of the mature DCs is recognized by T cell receptor (TCR). A critical step in DC-T activation is the proliferation of T cells. Therefore, DCs loaded with Seq8-Sf9 are co-cultured with CD4+ T cells labeled by CFSE fluorescent dye. After antigen presentation, T cells can rapidly proliferate and differentiate into Th cells (helper T cells). The immune conditions are thus evaluated based on the proliferation of CD4⁺ T cells. As shown in FIGS. 15a-15b, DCs from different healthy human beings loaded with Seq8-Sf9 can significantly promote the proliferation of T cells, which is positively correlated with the Seq5-Sf9 concentration. Under the same experimental conditions, Provenge exhibits an identical effect.

### 3.5.3 In vitro evaluation of antigen presentation of Seq8-Sf9-loaded DCs for activated cytotoxic T cells

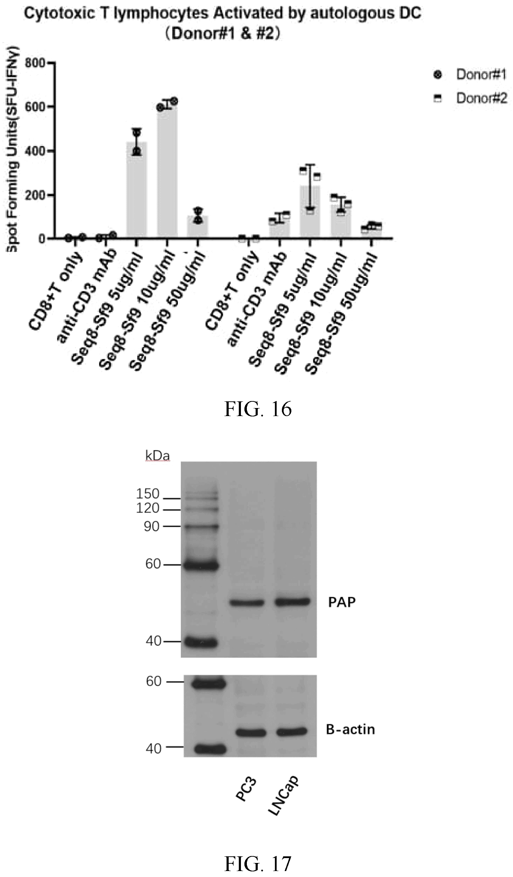

Antigen peptide/MHC class I complex presented on the DC membrane surface can be directly recognized by and bound to TCR on the surface of CD8+T cells, thereby activating CD8+T cells to play their biological effects, in which one of the main ways is the secretion of interferon-γ (IFN-γ). DCs loaded with Seq8-Sf9 are co-cultured with CD8+T cells from the same volunteer, and the Seq8-Sf9-loaded DCs are added again for secondary stimulation to activate a large amount of CD8⁺ T cells. IFN-γ secreted at a single-cell level is detected by ELISPOT. The detection result is shown in FIG. 16. It can be seen that the IFNγ of the Seq8-Sf9-loaded group is significantly more than that of the own CD8+T cells from volunteers (negative control), that is, Seq8-Sf9 can effectively promote the activation of CD8+T cells.

### 3.5.4 Evaluation of the killing effect of CTLs activated by Seq8-Sf9 using PAP antigen-expressing human prostate cancer cell lines as target cells

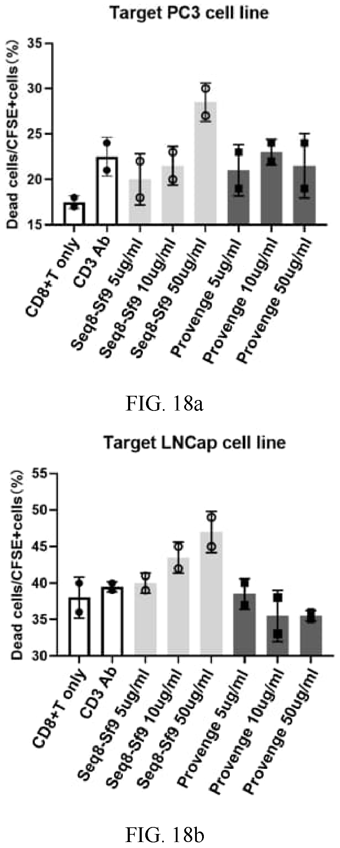

Treatment of prostate cancer exhibits different therapeutic effects depending on androgen sensitivity. PC3 is a common androgen-independent human prostate cancer cell line, while LNCaP is androgen-dependent. Both of above cell lines express a certain level of PAP, and as shown in FIG. 17, LNCaP expresses relatively more PAP protein. These cell lines are used as target cells and co-cultured with effector cells (CTLs activated by Seq8-Sf9-loaded DCs). The absolute count of dead cells is calculated by CFSE and Counting Beads. As shown in FIGS. 18a-18b, CTLs activated by Seq8-Sf9-loaded DCs can directly kill target cells, and the killing effect is proportional to the concentration of the Seq8-Sf9. In addition, the number of dead cells of LNCaP expressing higher amount of PAP cells is much higher than that of PC3 cells, verifying a targeted killing effect of the CTL activated by DCs loaded with the Seq8-Sf9, and the effect thereof being higher than that of Provenge.

### 3.6 Evaluation of Seq8-Sf9 immunogenicity

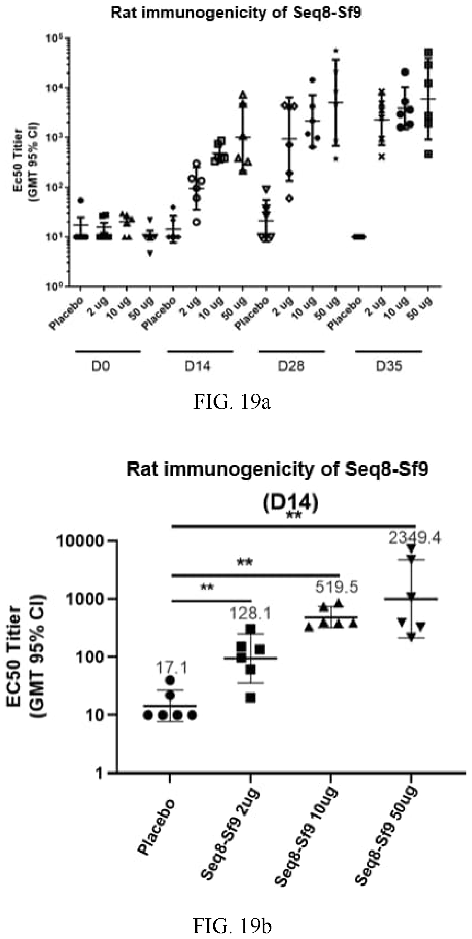

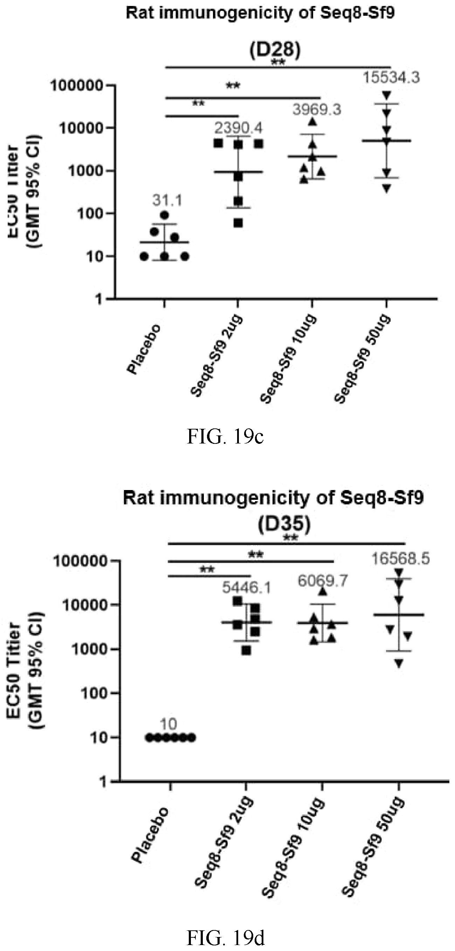

To evaluate effect of the immune response of Seq8-Sf9 in vivo, 6-8 week-old Sprague Dawley (SD) male rats are immunized with Seq8-Sf9. The SD rats are subcutaneously injected with Seq8-Sf9 every two weeks (placebo per rat, 2 µg per rat, 10 µg per rat, 50 µg per rat), for a total of three injections. The rat serum is collected (on Day 14, Day 28, and Day 35 (one week after the third immunization)) after the immunization of rats with Seq8-Sf9, in order to detect Seq8-Sf9-specific antibody titer. As shown in FIGS. 19a-19d, compared with the Placebo group, the vaccine-specific serum antibodies are detected at a low dose of 2 µg, and the antibody titer reaches the highest level at the third injection and one week after the fourth injection.

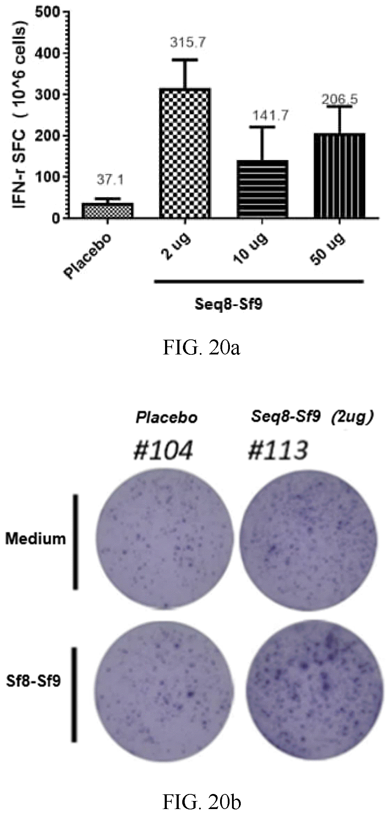

In addition, rat spleens are harvested on Day 35 and re-stimulated with Seq8-Sf9 in vitro to activate and induce the activation of effector T cells. IFN-γ secretion of the single spleen cell is detected by ELISPOT. As shown in FIGS. 20a-20b, splenic immune cells can rapidly differentiate into cytotoxic T lymphocytes to secrete IFN-γ, under the action of the secondary stimulation of Seq8-Sf9.

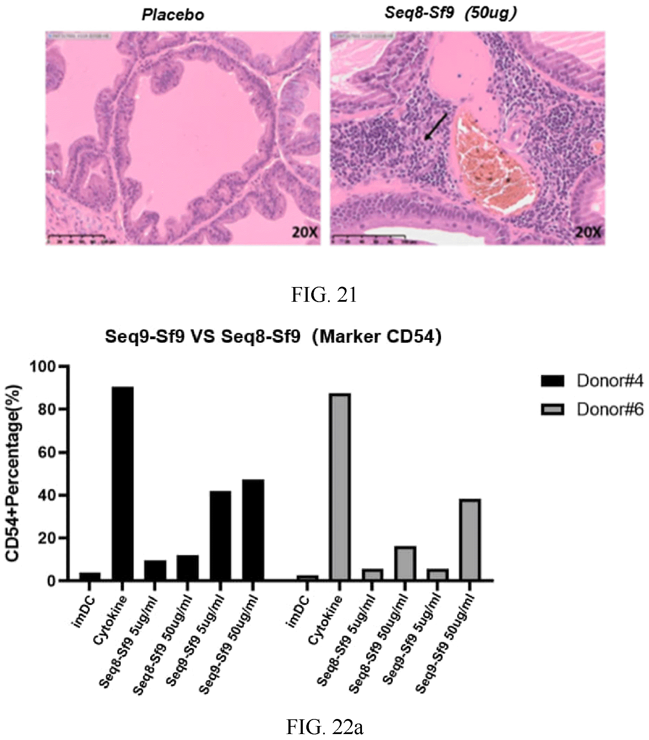

Meanwhile, the prostate tissue is observed and the observation result is shown in FIG. 21, indicating that inflammatory cell infiltration can also be observed in the prostate tissue of rats in the Seq8-Sf9 group.

In summary, Seq8-Sf9 can establish a complete in-vivo immune response in the SD rats and effectively activate T cells, i.e., Seq8-Sf9 can complete an in-vivo immune activation.

### 3.7 Evaluation of the immune activation effect of fusion protein Seq9-Sf9 using DCs induced by CD14⁺ monocytes in human peripheral blood PBMCs

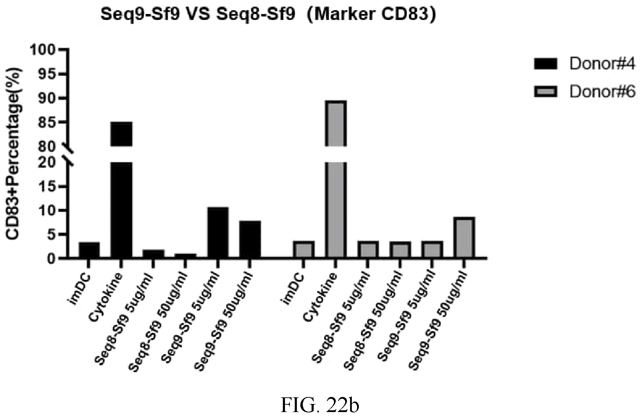

The Fc fragment of Seq9-Sf9 differs from that of Seq8-Sf9. To evaluate its immune activation effect, CD14⁺ monocytes sorted from PBMCs of two healthy human beings are induced to differentiate and loaded with Seq9-Sf9. Changes in surface markers of the antigen-loaded DCs are detected by flow cytometry. As shown in FIGS. 22a-22b, Seq9-Sf9 can increase the expression of CD54 and CD83 on the cell surface of DCs to promote activation of DCs, and has a higher expression level of marker than Seq8-Sf9.

Finally, it should be noted that, upon consideration of the specification and practice of the application disclosed herein, other embodiments of the application will be readily apparent to those skilled in the art. The present application is intended to cover any variations, uses, or adaptations of the application that follow its general principles and include common knowledge or conventional technical means in the technical field that are not disclosed herein and is not limited to the content set forth above; and various modifications and changes may be made without departing from the scope thereof. The scope of the application is limited only by the appended claims.

## Claims

1. A glycosyl-modified fusion protein, comprising a murine Fc variant and a hepatitis B virus antigen, wherein the fusion protein binds to and activates DC cells and promotes proliferation and activation of specific T cells;
the murine Fc variant is obtained by subjecting a murine Fc fragment to amino acid mutation and glycosylation modification;
the murine Fc variant comprises at least one of alanine at position 223, alanine at position 228, alanine at position 230, leucine at position 330, and glutamic acid at position 332;
in the glycosylation modification, a glycosyl is derived from at least one of mannose, N-acetylglucosamine, and fucose;
in the glycosylation modification, a saccharide chain structure formed by a linkage of the glycosyl is selected from at least one of a high-mannose type, an oligomannose type, and a fucose type;
the glycosylation modification does not comprise sialic acid modification;
a position numbering of amino acid is performed according to EU numbering system.

2. The fusion protein according to claim 1, wherein an amino acid sequence of the murine Fc variant is shown in SEQ ID NO:2 or SEQ ID NO:3.

3. The fusion protein according to claim 1 or 2, wherein the hepatitis B virus antigen is HBV S1 and/or HBV S2 and/or HBV CORE.

4. The fusion protein according to any one of claims 1-3, wherein the fusion protein further comprises a protein tag.

5. The fusion protein according to any one of claims 1-4, wherein the fusion protein further comprises a linker peptide.

6. A nucleic acid molecule encoding the fusion protein according to any one of claims 1-5.

7. A recombinant expression vector comprising the nucleic acid molecule according to claim 6.

8. A host cell comprising the recombinant expression vector according to claim 7.

9. A pharmaceutical composition comprising the fusion protein according to any one of claims 1-5 and a pharmaceutically acceptable carrier.

10. Use of the fusion protein according to any one of claims 1-5 in preparation of a medicament for treating hepatitis B virus and hepatitis B-associated hepatocellular carcinoma.

11. Use of the fusion protein according to any one of claims 1-5 in preparation of a medicament for treating any hepatitis B virus expressing HBV S1 and/or HBV S2 and/or HBV core protein antigens and hepatitis B-associated hepatocellular carcinoma.

12. A method for treating hepatitis B virus infection, wherein the fusion protein according to any one of claims 1-5 is administered to a subject.
